# EUROPÄISCHE PATENTSCHRIFT

(11) **EP 1 848 751 B1**
(45) Veröffentlichungstag und Bekanntmachung des Hinweises auf die Patenterteilung: **23.06.2010**
(21) Anmeldenummer: 05819245.1
(22) Anmeldetag: 15.12.2005
(51) Int. Cl.: C08F 220/06, C08F 220/18, A61K 9/00, A61K 9/28

(54) **TEILNEUTRALISIERTES ANIONISCHES (METH)ACRYLAT-COPOLYMER**
PARTLY NEUTRALISED ANIONIC (METH)ACRYLATE COPOLYMER
COPOLYMERE DE (METH)ACRYLATE ANIONIQUE PARTIELLEMENT NEUTRALISE

(30) Priorität: 15.02.2005 DE 102005007059
(43) Veröffentlichungstag der Anmeldung: 31.10.2007
(73) Patentinhaber: Evonik Röhm GmbH, 64293 Darmstadt (DE)
(72) Erfinder: LIZIO, Rosario, 64380 Rossdorf (DE); PETEREIT, Hans-Ulrich, 64291 Darmstadt (DE); ROTH, Erna, 64297 Darmstadt (DE); DAMM, Michael, 63322 Rödermark (DE); ALEXOWSKY, Rüdiger, 64569 Nauheim (DE)
(86) Internationale Anmeldenummer: PCT/EP2005/013513
(87) Internationale Veröffentlichungsnummer: WO 2006/087027

(56) Entgegenhaltungen:
- EP-A- 0 088 951
- US-A- 5 648 399

## Beschreibung

Die Erfindung betrifft ein teilneutralisiertes, anionisches (Meth)aaylat-Copolymer, eine damit überzogene Arzneiform, Verfahren zur Herstellung der Arzneiform und die Verwendung des teilneutralisiertes (Meth)acrylatcopolymeren zur Herstellung einer, den Wirkstoff bei einem bestimmten pH-Wert schnell freisetzenden Arzneiform.

### Stand der Technik

US 5,648,399 **beschreibt Verfahren zur Behandlung von Mycosen in der Mundhöhle unter Anwendung topischer Applikationen von flüssigen Polymerkompositionen, die einen geeigneten Wirkstoff enthalten.**

EP 0 088 951 A2 beschreibt ein Verfahren zum Überziehen von Arzneiformen mittels eines in Wasser dispergierten Überzugsmittels. Für die Redispergierung von Carboxylgruppen-haltigen (Meth)acrylatcopolymeren von Pulvern zu Dispersionen wird die Teilneutralisation der Carboxylgruppen empfohlen. Die Salzbildung der sauren Gruppen tritt durch Umsetzung mit einer Base ein. Als Basen kommen Alkalien, wie z.B. Natronlauge, Kalilauge, Soda, Pottasche, Natriumbikarbonat, Trinatriumphosphat, Trinatriumcitrat oder Ammoniak oder physiologisch verträgliche Amine, wie Triethanolamin oder Tris-(hydroxymethyl)-aminomethan, in Betracht. Günstig in Bezug auf die Redispergierung ist ein Neutralisationsgrad von 0,1 bis 10 Gew.-% der im Copolymeren enthaltenen Carboxylgruppen.

WO 2004/096185 beschreibt eine Arzneiform und ein Verfahren zu ihrer Herstellung. Die Arzneiform ist mit einem anionischen (Meth)acrylatcopolymeren überzogen, das bei Bedarf teilneutralisiert werden kann. Um eine Lösung des anionischen Copolymers herzustellen ist in der Regel eine teilweise oder vollständige Neutralisation der Säuregruppen nötig.

Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7.

### Aufgabe und Lösung

Anionische (Meth)acrylatcopolymere, z. B. vom Typ EUDRAGIT^{®} L, EUDRAGIT® L100-55, EUDRAGIT® S oder EUDRAGIT® FS, sind als darmsaftlösliche Überzüge für Arzneiformen bekannt. In Abhängigkeit von der Monomerzusammensetzung, insbesondere aber in Abhängigkeit von Gehalt anionischer Gruppen, sind die anionische (Meth)acrylatcopolymere durch spezifische Auflösungs-pH-Werte in Darmsaft oder in künstlichem Darmsaft charakterisiert. Je nach Polymer-Typ liegen die spezifischen Auflösungs-pH-Werte bzw. die pH-Werte des spezifischen Beginns der Auflösung im Bereich von z. B. pH 5,5 bis 7,5. Ab dem für das jeweilige anionische (Meth)acrylatcopolymere spezifischen Auflösungs-pH-Wert und darüber setzen damit überzogene Arzneiformen den enthaltenen Wirkstoff frei. Die spezifischen Auflösungs-pH-Werte charakterisieren somit den Beginn der Wirkstofffreisetzung.

Es ist bekannt anionische (Meth)acrylatcopolymere in teilneutralisierter Form einzusetzen. Dadurch wird eine verbesserte Löslichkeit des Polymeren in Wasser und eine Stabilisierung der Polymerdispersionen erreicht. Als Basen für die Teilneutralisation werden in der Regel Substanzen wie NaOH, KOH, Ammoniumhydroxyd oder organische Basen, wie z. B. Triethanolamin, empfohlen.

Vergleicht man z. B mittels NaOH teilneutralisierte und nicht teilneutralisierte Filme aus anionischen (Meth)acrylatcopolymere, so stellt man fest, dass sich die teilneutralisierten Filme schneller in einen Puffersystem bei ihrem spezifischen Auflösungs-pH-Wert auflösen als die nicht neutralisierten Filme.

Das gleiche gilt für Verwendung eines teilneutralisierten anionischen (Meth)acrylat-Copolymeren als Überzugsmittel für eine Arzneiform, die den enthaltenen Wirkstoff im Freigabetest nach USP 28 beim spezifischen pH-Wert des Beginns der Wirkstofffreisetzung schneller freisetzt als eine vergleichbare Arzneiform mit gleichem Polymerzug, jedoch ohne Teilneutralisation.

Für eine Reihe von Therapien wären Arzneiformen, die ein solches beschleunigtes Wirkstofffreigabeverhalten zeigen würden, das bei dem, für das eingesetzte (Meth)acrylatcopolymer spezifischen pH-Wert einsetzt, wünschenswert. Die Erfinder haben jedoch festgestellt, dass sich das oben beschriebene Verhalten teilneutralisierter Filme und mit teilneutralisierten Filmen überzogener Arzneiformen nicht oder nur vermindert einstellt, wenn die nach dem Stande der Technik bekannten Basen für die Teilneutralisation verwendet wurden und man die Filme oder Arzneiformen zunächst bei pH 1,2 für 2 Stunden belässt bevor man auf den spezifischen pH-Wert des Beginns der Wirkstofffreisetzung umpuffert. Gerade diese Bedingungen liegen jedoch in-vivo vor, wenn eine Arzneiform zunächst in den Magen gelangt und dann erst in den Darmtrakt befördert wird. Die bekannte Teilneutralisation von anionischen (Meth)acrylatcopolymeren ist daher nicht geeignet, um eine beschleunigtes Wirkstofffreisetzungsverhalten zu erreichen.

Es wurde daher als Aufgabe gesehen, anionische (Meth)acrylatcopolymere so zu formulieren, dass damit überzogene Arzneiformen, den enthaltenen Wirkstoff ab dem spezifischen Auflösungs-pH-Wert beschleunigt freigeben.

Die Aufgabe wird gelöst durch ein teilneutralisiertes anionisches (Meth)acrylat-Copolymer, bestehend aus radikalisch polymerisierten Einheiten von 25 bis 95 Gew.-% C₁- bis C₄-Alkylestem der Acryl- oder der Methacrylsäure und 5 bis 75 Gew.-% (Meth)acrylat-Monomeren mit einer anionischen Gruppe, wobei 0,1 bis 25 % der enthaltenen anionischen Gruppen mittels einer Base neutralisiert sind,
**dadurch gekennzeichnet, dass**
die Base **Lysin oder** eine kationische, organische Base mit einem Molekulargewicht über 150 ist.

### Ausführung der Erfindung

### Anionisches (Meth)acrylat-Copolymer

Die Erfindung betrifft ein teilneutralisiertes, anionisches (Meth)acrylat-Copolymer.

Das anionische (Meth)acrylat-Copolymere besteht zu 25 bis 95, bevorzugt zu 40 bis 95, insbesondere zu 60 bis 40 Gew.-% aus radikalisch polymerisierten C₁- bis C₄-Alkylestern der Acryl- oder der Methacrylsäure und zu 75 bis 5, bevorzugt 60 bis 5, insbesondere 40 bis 60 Gew.-% aus (Meth)acrylatMonomeren mit einer anionischen Gruppe.

In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein. Bevorzugt sind keine weiteren vinylisch copolymerisierbaren Monomere enthalten.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe ist z. B. Acrylsäure, bevorzugt ist Methacrylsäure.

Geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

EUDRAGIT® L ist ein Copolymer aus 50 Gew.-% Methylmethacrylat und 50 Gew.-% Methacrylsäure. Der pH-Wert des Beginns der spezifischen Wirkstofffreisetzung in Darmsaft oder künstlichem Darmsaft kann mit pH 6,0 angegeben werden.

EUDRAGIT® L100-55 ist ein Copolymer aus 50 Gew.-% Ethylacrylat und 50 Gew.-% Methacrylsäure. EUDRAGIT® L 30 D-55 ist eine Dispersion enthaltend 30 Gew.-% EUDRAGIT® L 100-55. Der pH-Wert des Beginns der spezifischen Wirkstofffreisetzung in Darmsaft oder künstlichem Darmsaft kann mit pH 5,5 angegeben werden.

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S). Der pH-Wert des Beginns der spezifischen Wirkstofffreisetzung in Darmsaft oder künstlichem Darmsaft kann mit pH 7,0 angegeben werden.

Geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS). Der pH-Wert des Beginns der spezifischen Wirkstofffreisetzung in Darmsaft oder künstlichem Darmsaft kann mit pH 7,0 angegeben werden.

EUDRAGIT® FS ist ein Copolymer aus 25 Gew.-%, Methylmethacrylat, 65 Gew.-% Methylacrylat und 10 Gew.-% Methacrylsäure. EUDRAGIT® FS 30 D ist eine Dispersion enthaltend 30 Gew.-% EUDRAGIT® FS.

Weiterhin geeignet ist ein Copolymer, welches sich aus
20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
zusammensetzt, mit der Maßgabe, daß die Glastemperatur des Copolymers nach ISO 11357-2, Punkt 3.3.3, höchstens 60 °C beträgt. Dieses (Meth)acrylatcopolymer ist wegen seiner guten Reißdehnungseigenschaften insbesondere zum Verpressen von Pellets zu Tabletten geeignet.

Weiterhin geeignet sind Copolymere aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und gegebenenfalls
0 bis 10 Gew.-% weiteren vinylisch copolymerisierbarer Monomeren,
wobei sich die Anteile der Monomeren zu 100 Gew.-% addieren, zusammensetzt, mit der Maßgabe, daß die Glastemperatur des Copolymers (glass transition temperature) nach ISO 11357-2, Punkt 3.3.3 (midpoint temperature *T*_{mg}), 55 bis 70 °C beträgt. Copolymere dieses Typs sind wegen
seiner guten mechanischen Eigenschaften insbesondere zum Verpressen von Pellets zu Tabletten geeignet.

Das oben genannte Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 25 Gew.-% Methylacrylat,
20 bis 40, bevorzugt 25 bis 35, besonders bevorzugt 18 bis 22 Gew.-% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew.-%, aus den Monomeren Methacrylsäure, Methylacrylat, Ethylacrylat und Butylmethacrylat in den oben angegebenen Mengenbereichen.

Es können jedoch zusätzlich, ohne daß dies zu einer Beeinträchtigung der wesentlichen Eigenschaften führen muß, geringe Mengen im Bereich von 0 bis 10, z. B. 1 bis 5 Gew.-% weiterer vinylisch copolymerisierbarer Monomere, wie z. B. Methylmethacrylat, Butylacrylat, Hydroxyethylmethacrylat, Vinylpyrrolidon, Vinylmalonsäure, Styrol, Vinylalkohol, Vinylacetat und/oder deren Derivate enthalten sein.

### Herstellung der anionischen (Meth)acrylatcopolymere

Die Herstellung der anionischen (Meth)acrylatcopolymere kann in an sich bekannter Weise durch radikalische Polymerisation der Monomeren erfolgen (siehe z. B. EP 0 704 207 A2 und EP 0 704 208 A2). Das erfindungsgemäße Copolymer ist in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.

Das Copolymerisat kann nach gängigen Verfahren der radikalischen Polymerisation kontinuierlich oder diskontinuierlich (Batch-Verfahren) in Gegenwart radikalbildender Initiatoren und gegebenenfalls Reglern zur Einstellung des Molekulargewichts in Substanz, in Lösung, durch Perlpolymerisation oder in Emulsion hergestellt werden. Das mittlere Molekulargewicht Mw (Gewichtsmittel, bestimmt z. B. durch Messung der Lösungsviskosität) kann z. B. im Bereich von 80.000 bis 1.000.000 (g/mol) liegen. Bevorzugt ist die Emulsionspolymerisation in wäßriger Phase in Gegenwart wasserlöslicher Initiatoren und (vorzugsweise anionischer) Emulgatoren.
Im Falle der Substanzpolymerisation kann das Copolymer in fester Form durch Brechen, Extrusion, Granulieren oder Heißabschlag erhalten werden.

Die (Meth)acrylatcopolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Teilneutralisation

### Eine geeignete Base ist Lysin.

Geeignete kationische, organische Basen mit einem M_{w} > 150, bevorzugt > 155, besonders bevorzugt > 160, z. B. von > 150 bis 20.000 sind:
Die kationischen, basischen Animosäuren Histidin und/oder Arginin Die Aminosäuren Glutamin und Asparagin sind nicht geeignet, da sie eine nicht protonierte Säureamidfunktion aufweisen und somit nicht den kationischen Basen zuzurechnen sind.
Natürliche oder synthetische Oligomere oder Polymere, z, B. aus 3 bis 100, bevorzugt 5 bis 25 Einheiten, von Histidin, Arginin oder Lysin, Poly-Histidine, PolyArginine, Poly-Lysine,
kationische bzw. zwitterionische Phopholipide, wie z. B. Phosphatidylcholin,
Ribonukleoside: Kondensationsprodukte der Hydroxylfunktion am Kohlenstoffatom 1 der Ribose mit der heterocyklischen Aminofunktion der Basen Adenin, Guanin, Cytosin, Thymin oder Uracil, entsprechend dem Vorkommen in der RNA
Desoxyribonukleoside: Kondensationsprodukte der Hydroxylfunktion am Kohlenstoffatom 1 der Desoxyribose mit der heterocyklischen Aminofunktion der Basen Adenin, Guanin, Cytosin, Thymin oder Uracil, entsprechend dem Vorkommen in der DNA.
Basen aus kationischen oberflächenaktiven Hilfsstoffen oder Emulgatoren, wie Benzalkonium ( CAS RN: 8001-54-5), Benzethonium (CAS 121-54-0), Cetalkonium ( CAS 122-18-9), Cetrimide ( CAS 8044-71-1), Cetrimonium ( CAS 57-09-0), Cetylpyridinium (CAS 123-03-5), Stearalkonium (CAS 122-19-0), Diallyldimethylammonium (CAS 230-993-8)
Nicht geeignet für die Zwecke der Erfindung sind Basen, die in EP 0 088 951 A2 oder WO 2004/096185 ausdrücklich genannt oder daraus ableitbar sind. Insbesondere sind ausgeschlossen: Natronlauge, Kalilauge (KOH) Ammoniumhydroxyd oder organische Basen wie z. B. Triethanolamin, Soda, Pottasche, Natriumbikarbonat, Trinatriumphosphat, Trinatriumcitrat oder Ammoniak oder physiologisch verträgliche Amine, wie Triethanolamin oder Tris-(hydroxymethyl)-aminomethan.

Diese Basen weisen ein Mw von höchstens 150 auf (Triethanolamin). Obwohl Triethanolamin mit seinem Molekulargewicht nahe bei den Aminosäuren Histidin, Arginin, Lysin liegt tritt der erfindungsgemäße Effekt mit dieser Substanz nicht bzw. nur unzureichend ein. Trinatriumphosphat, Trinatriumcitrat sind nicht kationischer Natur, sondern Salze der entsprechenden Säuren. Ammoniumhydroxyd, Natronlauge, Kalilauge (KOH), Soda, Pottasche, Natriumbikarbonat weisen nur geringe Molekulargewichte auf bzw. sind den anorganischen Basen zuzurechnen.

Das Molekulargewicht der genannten Substanzen ist bekannt bzw. kann anhand der in Molekül vorhandenen Atome anhand der Atomgewichte errechnet werden.

### Einstellung des Teilneutrallsationsgrades durch Mischungen

Verfahrenstechnische Vorteile bei der Einstellung des Teilneutralisationsgrades können sich auch durch Mischungen ergeben. Die Erfindung betrifft Mischungen von anionischen (Meth)acrylat-Copolymeren mit unterschiedlichem Teilneutrallsationsgrad, bestehend aus radikalisch polymerisierten Einheiten von 25 bis 95 Gew.% C₁- bis C₄-Alkylestem der Acryl- oder der Methacrylsäure und 5 bis 75 Gew.-% (Meth)acrylat-Monomeren mit einer anionischen Gruppe,
dadurch gekennzeichnet, dass im rechnerischen Durchschnitt der Mischung 0,1 bis 25 % der enthaltenen anionischen Gruppen mittels einer Base neutralisiert sind, die Lysin oder eine kationische, organische Base mit einem Molekulargewicht über 150 ist. Es ist beispielsweise möglich ein nicht teilneutratisiertes, anionisches (Meth)acrylat-Copolymer, bestehend aus radikalisch polymerisierten Einheiten von 25 bis 95 Gew.-% C₁- bis C₄-Alkylestem der Acryl- oder der Methacrylsäure und 5 bis 75 Gew.-% (Meth)acrylat-Monomeren mit einer anionischen Gruppe mit einem teilneutralisierten (Meth)acrylatcopolymeren gleicher Monomerzusammensetzung innerhalb der genannten mengenmäßigen Bereiche zu mischen, so dass im rechnerischen Durchschnitt der Mischung 0,1 bis 25 % der enthaltenen anionischen Gruppen neutralisiert sind.
Die Mischung kann z. B. hergestellt werden, indem in eine Dispersion eines nicht teilneutralisierten, anionischen (Meth)acrylat-Copolymeren ein Pulver eingerührt wird, das aus einer Dispersion eines teilneutralisierten, anionischen (Meth)acrylat-Copolymeren, z. B. durch Sprüh- oder Gefriertrocknung, gewonnen wurde.

Die kationische, organische Base mit einem Molekulargewicht Ober 150 ist folgend dem Prinzip der vorliegenden Erfindung wiederum z. B. Histidin, Arginin, ein Poly-Histidin, ein Poly-Arginin, ein Polylysin, ein Desoxyribonukleosid, eine Base aus kationischen oberflächenaktiven Hilfsstoffen oder Emulgatoren.

### Mischungen

Das erfindungsgemäß teilneutralisierte (Meth)acrylat-Copolymer eignet sich weiterhin zur Mischung mit anderen pharmazeutisch genutzten Copolymeren, um deren Eigenschaften zu modifizieren. Dies erhöht die Gestaltungsfreiräume des Fachmanns bei der Einstellung speziell modifizierter Freigabeprofile.
Die Erfindung betrifft demnach ein teilneutralisiertes (Meth)acrylat-Copolymer, dadurch gekennzeichnet, dass es in Mischung vorliegt mit Copolymeren aus Methylmethacrylat und/oder Ethylacrylat und gegebenenfalls weniger als 5 Gew.-% Methacrylsäure, Copolymeren aus Methylmethacrylat, Butylmethacrylat und Dimethylethylmethacrylat, Copolymeren aus Methylmethacrylat, Ethylacrylat und Trimethylammoniumethylmethacrylat, Copolymeren aus Methylmethacrylat und Ethylacrylat, Polyvinylpyrolidonen (PVP), Polyvinylalkoholen, Polyvinylalkohol-Polyethylenglycol-Graft-Copolymeren (Kollicoat®), Stärke und deren Derivaten, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsäure-Copolymer 9:1 (VAC : CRA, Kollicoat® VAC), Polyethylenglykolen mit einem Molekulargewicht über 1000 (g/mol), Chitosan, einer vernetzten und/oder unvernetzten Polyacrylsäure, einem Na-Alginat, und/oder ein Pektin.

### Dispersionen

Das teilneutralisierte (Meth)acrylat-Copolymer kann z. B. in Form einer wäßrigen Dispersion mit 10 bis 50-prozentigen Feststoffanteil vorliegen.

Das teilneutralisierte (Meth)acrylat-Copolymer kann in Form eines redispergierbaren Pulvers vorliegen, welches aus einer Dispersion z. B. durch Sprühtrocknung gewonnen wurde.

### Dispersionen / Teilneutralisation

Das Emulsionspolymerisat wird vorzugsweise in Form einer 10- bis 50-gew.prozentigen, insbesondere 20 bis 40-prozentigen wäßrigen Dispersion erzeugt und angewendet. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert Latex-Teilchengröße (Radius) beträgt in der Regel 40 bis 100 nm, vorzugsweise 50 bis 70 nm, was eine verarbeitungstechnisch günstige Viskosität unter 1000 mPa · s gewährleistet. Die Teilchengröße kann durch Laserbeugung, z. B. mit dem Mastersizer 2000 (Fa. Malvern), bestimmt werden.

Bei höheren Neutralisationsgrades z. B. 10 bis 50 Mol.-% oder vollständiger Neutralisation ist es möglich, das Copolymer in einen gelösten Zustand zu überführen.

Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer erfindungsgemäßen basischen Substanz wie z. Lysin oder Arginin. Es ist auch möglich ein Pulver des Copolymeren einzusetzen, dem bereits bei seiner Herstellung zum Zweck der (Teil)neutralisation eine Base z. B. Lysin zugesetzt wurde, so daß das Pulver ein bereits (teil)neutralisiertes Polymer ist. Der pH-Wert der Lösung liegt in der Regel über 4, z. B. im Bereich von 4 bis ca. 7. Man kann dabei auch z. B. Mischungen von Batches von voll- oder teilneutralisierten Dispersionen mit nicht neutralisierten Dispersionen vornehmen und in beschriebener Weise weiterverarbeiten, d. h. die Mischung für Überzüge verwenden oder zunächst zu einem Pulver gefrier- oder sprühtrocknen.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation und Abquetschen des Wassers in einem Extruder mit anschließender Granulation (siehe z. B. EP-A 0 683 028).

Copolymer-Dispersionen aus sprüh- oder gefriergetrockneten und redispergierten Pulvern können eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10; bevorzugt zu 5 bis 7 Mol-% in teilneutralisierter Form vorliegt (bezogen auf die im Copolymer enthaltenen Säuregruppen). Bevorzugt ist zu diesem Zweck die Teilneutralisation mittels Zugabe von Lysin oder Arginin. Bevorzugt ist ein anionischer Emulgator in Menge von 0,1 bis 2 Gew.-% enthalten. Besonders bevorzugt ist Natriumlaurylsulfat als Emulgator.

### Verwendung des teilneutralisierten (Meth)acrylatcopolymeren

Das teilneutralisierte, anionische (Meth)acrylat-Copolymere kann als Überzugsmittel verwendet werden für eine Arzneiform, die 90 %, bevorzugt 95 oder 100 % des enthaltenen Wirkstoff im Freigabetest nach USP 28 für 2 Stunden bei pH 1,2 und anschließendem Umpuffern auf den pH-Wert des Beginns der Wirkstofffreisetzung in höchstens 90 %, bevorzugt höchstens 75 %, insbesondere höchstens 50 % der Zeit freisetzt, die bei einer vergleichbaren Arzneiform mit gleichem Polymerzug, jedoch ohne oder Teilneutralisation mittels anderer, nicht erfindungsgemäßer Basen, dafür verstreicht.

Setzt die nicht erfindungsgemäße Arzneiform den Wirkstoff im Freigabetest nach USP 28 für 2 Stunden bei pH 1,2 und anschließendem Umpuffern auf den pH-Wert des Beginns der Wirkstofffreisetzung, z.B. pH 5,5, in z. B. 120 min zu 90 % frei, benötigt eine vergleichbare erfindungsgemäße Arzneiform dafür höchstens 108 min (90 % der Zeit), höchstens 90 min (75 %) oder höchstens 60 min (50 %).

Dem Fachmann ist der Freisetzungstest nach USP 28, insbesondere nach USP 28 <711> Paddle-Methode (= Apparatus 2), hinlänglich bekannt

Der typische Testablauf ist der Folgende:
1. Die Gefäße der Freisetzungsaparatur werden mit je 360mL 0,1M-HCl (pH 1,2) gefüllt und die Temperatur des Wasserbads auf 37 ± 0,5°C eingestellt.
2. Der Blattrührer wird mit einer Umdrehungsrate von 100rpm angeschaltet.
3. In jedes Gefäß der Apparatur werden 1g Pellets gegeben. Es wird darauf geachtet, dass keine Luftblasen auf der Pellet-Oberfläche sind.
4. Nach 120 min werden 140mL Phosphat-Pufferlösung (auf 37°C temperiert) zugesetzt, so dass im Endvolumen von 500mL der gewünschte pH-Wert entsteht: pH 5,5; 5,6; 5,7; 5,8 oder 7,0.
5. Bestimmung des Zeitpunktes der 100%-igen Wirkstofffreigabe, je nach Wirkstoff, z. B. im Falle von Theophyllin photometrisch bei 271 nm, im Umlaufverfahren.

### Arzneiform

Die Erfindung betrifft eine Arzneiform, enthaltend einen Kern mit einem pharmazeutischen Wirkstoff und einen Polymerüberzug aus einem teilneutralisierten (Meth)acrylat-Copolymer.

Die Arzneiform kann bevorzugt einen Polymerüberzug mit Lysin oder Arginin als Neutralisationsmittel in Kombination mit 5 bis 25 Gew.-% eines Weichmachers enthalten.

Die entsprechende Arzneiform kann z. B. in Form einer multipartikulären Arzneiform, pellethaltigen Tabletten, Minitabletten, Kapseln, Sachets, Brausetabletten oder Trockensäften vorliegen.

### Trennschichten

Die Arzneiform kann bevorzugt zwischen dem wirkstoffhaltigen Kern und dem Polymerüberzug eine Schicht, enthaltend gegebenenfalls ein Bindemittel und eine kationische, organische Base mit einem Molekulargewicht über 150 aufweisen. Dies bietet in einzelnen Fällen den Vorteil, dass durch die Oberfläche der Arzneiform die Wirkstoff abgegebene Base von innen wieder nachgeliefert wird. Mit diesem Aufbau kann die Wirkstofffreisetzung nochmals beschleunigt werden.

Die Arzneiform kann zwischen dem Kern mit einem pharmazeutischen Wirkstoff und dem Polymerüberzug eine Trennschicht aufweisen. Die Trennschicht kann vorteilhafterweise zum Zwecke der Verhinderung von Interaktionen zwischen Bestandteilen des Kerns und des Polymerüberzugs dienen. Die Trennschicht kann aus inerten Filmbildnern (z.B. HPMC, HPC oder (Meth)acrylsäure-Copolymeren) oder z.B. Talkum oder anderen geeigneten pharmazeutischen Substanzen bestehen. Ebenso können Kombinationen aus Filmbildnern und Talkum oder ähnlichen Stoffen verwendet werden.

### Verfahren zur Herstellung einer Arzneiform

Die Erfindung betrifft weiterhin ein Verfahren zur Herstellung der erfindungsgemäßen Arzneiform in an sich bekannter Weise mittels pharmazeutisch üblicher Verfahren, wie direktem Verpressen, Verpressen von Trocken-, Feucht- oder Sintergranulaten, Extrusion und anschließende Ausrundung, feuchte oder trockene Granulation oder direkte Pelletierung oder durch Binden von Pulvern (Powder layering) auf wirkstofffreie Kugeln bzw. neutrale Kerne (Nonpareilles) oder wirkstoffhaltige Partikeln und mittels Auftrag des Polymerüberzugs im Sprühverfahren oder durch Wirbelschichtgranulation.

### Herstellung multipartikulärer Arzneiformen

Die Erfindung eignet sich insbesondere zur Herstellung multipartikulärer Arzneiformen, da das erfindungsgemäße Copolymer den hohen Drücken beim Verpressen der Pellets mit dem Füllstoff standhält.

Die Herstellung von multipartikulären Arzneiformen durch Verpressen eines pharmazeutisch üblichen Bindemittels mit wirkstoffhaltigen Partikeln ist z. B. Beckert ef al. (1996), "Compression of enteric-coated pellets to disintegrating tablets", International Journal of Pharmaceutics 143, S. 13 - 23*,* und in WO 96/01624 ausführlich beschrieben.

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23*)*. Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder anderen geeigneten pharmazeutischen Substanzen bestehen. Ebenso können Kombinationen aus Filmbildnern und Talkum oder ähnlichen Stoffen verwendet werden.

Es ist auch möglich eine Trennschicht aus teilweise bzw. vollneutralisierten Copolymer-Dispersionen aufzubringen.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikel erforderlich sein. Durch Vormischung der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipres^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

### Weitere Herstellungsverfahren für die erfindungsgemäße Arzneiform

Auftragsverfahren erfolgt mittels Sprühauftrag aus organischer Lösung, oder bevorzugt wäßrigen Dispersionen durch Schmelzen oder durch direkten Pulverauftrag. Für die Ausführung ist dabei entscheidend, daß gleichmäßige, porenfreie Überzüge entstehen.

Auftragsverfahren gemäß Stand der Technik s. z.B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation sind relevante Eigenschaften, geforderte Tests und Spezifikationen in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### BEISPIELE

### Freisetzungstest der Theophyllin-Pellets nach USP 28 <711> Paddle-Methode (= Apparatus 2)

Ablauf:
1. Die Gefäße der Freisetzungsaparatur werden mit je 360mL 0,1M-HCl (pH 1,2) gefüllt und die Temperatur des Wasserbads auf 37 ± 0,5°C eingestellt.
2. Der Blattrührer wird mit einer Umdrehungsrate von 10rpm angeschaltet.
3. In jedes Gefäß der Apparatur werden 1 g Pellets gegeben. Es wird darauf geachtet, dass keine Luftblasen auf der Pellet-Oberfläche sind.
4. Nach 120 min werden 140mL Phosphat-Pufferlösung (auf 37°C temperiert) zugesetzt, so dass im Endvolumen von 500mL der gewünschte pH-Wert entsteht: pH 5,5; 5,6; 5,7; 5,8 oder 7,0.
5. Bestimmung des Zeitpunktes der 100- igen Wirkstofffreigabe (photometrisch bei 271 nm, im Umlaufverfahren). Ergebnisse siehe Tabelle 1.

**Tabelle 1**

| | Theophyllin Pellets mit 30%-igen Überzug aus einem Copolymer aus 50 Gew.-% Ethylacrylat und 50 Gew.-% Methacrylsäure (EUDRAGIT® L 30 D-55), | | | | | |
|---|---|---|---|---|---|---|
| | 90 % Wirkstoff freigesetzt [min], Verfahren nach USP 28 Paddle, | | | | | |
| | Beispiel 1 | | Beispiel 2 | | Beispiel 3 | |
| | 15 %Teilneutralisation mit Lysin | | 15 %Teilneutralisation mit NaOH | | keine Teilneutralisation | |
| vorher 2h pH 1,2 | + | - | + | - | + | - |
| pH 5,5 | 45 | 40 | 90 | 45 | 120 | 120 |
| pH 5,6 | 30 | 28 | 50 | 30 | 60 | 60 |
| pH 5,7 | 20 | 19 | 30 | 20 | 50 | 50 |
| pH 5,8 | 18 | 17 | 20 | 18 | 30 | 30 |
| pH 7,0 | Sofortige Wirkstofffreisetzung | | | | | |

### Beispiel 1. Rezeptur mit Lysin

### Pelletüberzüge mit EUDRAGIT L 30 D 55 teilneutralisiert mit Lysin.

Auf 100 g Theophyllin Pellets, der Firma Klinge Pharma, mit der Teilchengröße von 0,7 -1,0 mm werden 30 % Trockensubstanz einer Polymerdispersion (Methacrylatcopolymer aus 50 Gew. - % Methacrylsäure und 50 Gew. - % Ethylacrylat) mit folgenden Rezepturen überzogen. Der Gesamttrockengehalt-Auftrag beträgt 35,7 Gew. % bezogen auf Ansatzmenge Freigabeuntersuchung 90 Gew.- % des Wirkstoffes siehe Tabelle 1.

| Materialien | (g) |
|---|---|
| EUDRAGIT L 30 D-55 | 100,00 |
| Lysin | 3,69 |
| Glycerinmonostearat | 1,50 |
| Polysorbat 80 | 0,60 |
| Wasser dem. | 132,81 |
| Summe | 238,60 |

### Sprühparameter im Hüttlin Mycrolab:

| | |
|---|---|
| Sprühdüse | 0,6 mm |
| Sprührate | 26 g/min/kg |
| Sprühdruck | 1,0 bar |
| Microklima | 0,6 bar |
| Zuluft Airflow | 20 m³ |
| Zulufttemperatur | 33 -39 °C |
| Produkttemperatur | 26 -29 °C |
| Machtrocknungszeit im Gerät | 10 min bei 40 °C |
| Sprühzeit | 1,5 - 2 h |
| Trocknung über Nacht bei | Raumtemperatur (RT) |

### Beispiel 2. Rezeptur mit NaOH

### Pelletüberzug mit EUDRAGIT L 30 D 55 teilneutralisiert mit NaOH.

Auf 100 g Theophyllin Pellets, der Firma Klinge Pharma, mit der Teilchengröße von 0,7 -1,0 mm werden 30 % Trockensubstanz einer Polymerdispersispersion (Methacrylatcopolymer aus 50 Gew. - % Methacrylsäure und 50 Gew. - % Ethylacrylat) mit folgenden Rezepturen überzogen. Der Gesamttrockengehalt - Auftrag beträgt 33,11 Gew. % bezogen auf Ansatzmenge Freigabeuntersuchung 90 Gew.- % des Wirkstoffes siehe Tabelle 1.

| Materialien | (g) |
|---|---|
| EUDRAGIT L 30 D-55 | 100,00 |
| Na OH | 1,01 |
| Glycerin monostearat | 1,50 |
| Polysorbat 80 | 0,60 |
| Wasser dem. | 117,62 |
| Summe | 220,73 |

### Sprühparameter im Hüttlin Mycrolab:

| | |
|---|---|
| Sprühdüse | 0,6mm |
| Sprührate | 27g/min/kg |
| Sprühdruck | 1,0 bar |
| Microklima | 0,6 bar |
| Zuluft Airflow | 20 m³ |
| Zulufttemperatur | 33 - 40 °C |
| Produkttemperatur | 26 - 30 °C |
| Machtrocknungszeit im Gerät | 10 min bei 40 °C |
| Sprühzeit | 1 - 1,5 h |
| Trocknung über Nacht bei RT | |

### Beispiel 3. Rezeptur ohne Teilneutralisierung

### Pelletüberzug mit EUDRAGIT L 30 D 55 ohne Teilneutralisierung.

Auf 100 g Theophyllin Pellets, der Firma Klinge Pharma, mit der Teilchengröße von 0,7 -1,0 mm werden 30 % Trockensubstanz einer Polymerdispersispersion (Methacrylatcopolymer aus 50 Gew. - % Methacrylsäure und 50 Gew. - % Ethylacrylat) mit folgenden Rezepturen überzogen. Der Gesamttrockengehalt - Auftrag beträgt 32,111 Gew. % bezogen auf Ansatzmenge. Freigabeuntersuchung 90 Gew.- % des Wirkstoffes siehe Tabelle 1.

| Materialien | (g) |
|---|---|
| EUDRAGIT L 30 D-55 | 100,00 |
| Glycerin monostearat | 1,50 |
| Polysorbat 80 | 0,60 |
| Wasser dem. | 136,83 |
| Summe | 238,93 |

### Sprühparameter im MiniGlatt:

| | |
|---|---|
| Sprühdüse | 0,5 mm |
| Sprührate | 1 - 2 g/min |
| Sprühdruck | 0,8 bar |
| Zuluft | 0,7 bar |
| Zulufttemperatur | 35 - 37 °C |
| Produkttemperatur | 32 - 33 °C |
| Machtrocknungszeit im Gerät | 10 min bei 40 °C |
| Sprühzeit | ca. 2 - 3 h |
| Trocknung über Nacht bei | RT |

## Patentansprüche

1. Arzneiform, enthaltend einen Kern mit einem pharmazeutischen Wirkstoff und einen Polymerüberzug aus einem teilneutralisierten (Meth)acrylat-Copolymer, bestehend aus radikalisch polymerisierten Einheiten von 25 bis 95 Gew.-% C₁- bis C₄-Alkylestern der Acryl- oder der Methacrylsäure und 5 bis 75 Gew.-% (Meth)acrylat-Monomeren mit einer anionischen Gruppe, wobei 0,1 bis 25 % der enthaltenen anionischen Gruppen mittels einer Base neutralisiert sind,
**dadurch gekennzeichnet, dass**
die Base Lysin oder eine kationische, organische Base mit einem Molekulargewicht über 150 ist.

2. Arzneiform, nach Anspruch 1, **dadurch gekennzeichnet, dass** die kationische, organische Base mit einem Molekulargewicht über 150 Histidin, Arginin, ein Poly-Histidin, ein Poly-Arginin, ein Poly-Lysin, ein Phopholipid, wie Phosphatidylcholin, ein Ribonukleosid oder ein Desoxyribonukleosid, eine Base aus kationischen oberflächenaktiven Hilfsstoffen oder Emulgatoren ist.

3. Arzneiform nach Anspruch 1 oder 2, **dadurch gekennzeichnet, dass** das anionische (Meth)acrylat Copolymer aus radikalisch polymerisierten Einheiten von 40 bis 60, Gew.-% Methacrylsäure und 60 bis 40 Gew.-% Methylmethacrylat oder 60 bis 40 Gew.-% Ethylacrylat besteht.

4. Arzneiform nach einem oder mehreren der Ansprüche 1 bis 3, **dadurch gekennzeichnet, dass** das anionische (Meth)acrylat Copolymer in Mischung vorliegt mit Copolymeren aus Methylmethacrylat und/oder Ethylacrylat und gegebenenfalls weniger als 5 Gew.-% Methacrylsäure, Copolymeren aus Methylmethacrylat, Butylmethacrylat und Dimethylethylmethacrylat, Copolymeren aus Methylmethacrylat, Ethylacrylat und Trimethylammoniumethylmethacrylat, Copolymeren aus Methylmethacrylat und Ethylacrylat, Polyvinylpyrolidonen (PVP), Polyvinylalkoholen, Polyvinylalkohol-Polyethylenglycol-Graft-Copolymeren (Kollicoat®), Stärke und deren Derivaten, Polyvinylacetatphtalat (PVAP, Coateric®), Polyvinylacetat (PVAc, Kollicoat), Vinylacetat-Vinylpyrolidon-Copolymer (Kollidon® VA64), Vinylacetat : Crotonsäure-Copolymer 9:1 (VAC : CRA, Kollicoat® VAC), Polyethylenglykolen mit einem Molekulargewicht über 1000 (g/mol), Chitosan, einer vernetzten und/oder unvernetzten Polyacrylsäure, einem Na-Alginat, und/oder ein Pektin.

5. Arzneiform nach einem oder mehreren der Ansprüche 1 bis 4, **dadurch gekennzeichnet, dass** der Polymerüberzug Lysin oder Arginin als Neutralisationsmittel im Kombination mit 5 bis 25 Gew.-% eines Weichmachers enthält.

6. Arzneiform nach einem oder mehreren der Ansprüche 1 bis 5, **dadurch gekennzeichnet, dass** sich zwischen dem wirkstoffhaltigen Kern und dem Polymerüberzug eine Schicht, enthaltend gegebenenfalls ein Bindemittel und eine kationische, organische Base mit einem Molekulargewicht über 150, befindet.

7. Arzneiform nach einem oder mehreren der Ansprüche 1 bis 5, **dadurch gekennzeichnet, dass** zwischen dem Kern mit einem pharmazeutischen Wirkstoff und dem Polymerüberzug eine Trennschicht aufgebracht ist.

8. Verfahren zur Herstellung einer Arzneiform, nach einem oder mehreren der Ansprüche 1 bis 7, **enthaltend einen Kern mit einem pharmazeutischen Wirkstoff und einen Polymerüberzug aus einem teilneutralisierten anionischen (Meth)acrylat-Copolyrner,** in an sich bekannter Weise mittels pharmazeutisch üblicher Verfahren, wie direktem Verpressen, Verpressen von Trocken-, Feucht- oder Sintergranulaten, Extrusion und anschließende Ausrundung, feuchte oder trockene Granulation oder direkte Pelletierung oder durch Binden von Pulvern (Powder layering) auf wirkstofffreie Kugeln bzw. neutrale Kerne (Nonpareilles) oder wirkstoffhaltige Partikeln und mittels Auftrag des Polymerüberzugs im Sprühverfahren oder durch Wirbelschichtgranulation.

9. Verfahren nach Anspruch 8, **dadurch gekennzeichnet, dass** das teilneutralisierte anionische (Meth)acrylat-Copolymer in Form einer wässrigen Dispersion mit 10 bis 50-prozentigen Feststoffanteil eingesetzt wird.

10. Verfahren nach Anspruch 8, **dadurch gekennzeichnet, dass** das teilneutralisierte anionische (Meth)acrylat-Copolymer in Form eines redispergierbaren Pulvers eingesetzt wird, welches aus einer Dispersion nach Anspruch 9 gewonnen wurde.

11. Verwendung eines teilneutralisierten anionisches (Meth)acrylat-Copolymeren, bestehend aus radikalisch polymerisierten Einheiten von 25 bis 95 Gew.-% C₁- bis C₄-Alkylestem der Acryl- oder der Methacrylsäure und 5 bis 75 Gew.-% (Meth)acrylat-Monomeren mit einer anionischen Gruppe, wobei 0,1 bis 25 % der enthaltenen anionischen Gruppen mittels einer Base neutralisiert sind,
**dadurch gekennzeichnet, dass**
die Base Lysin oder eine kationische, organische Base mit einem Molekulargewicht über 150 ist, als Überzugsmittel für eine Arzneiform, die 90 % des enthaltenen Wirkstoffs im Freigabetest nach USP 28 für 2 Stunden bei pH 1,2 und anschließendem Umpuffern auf den pH-Wert des Beginns der Wirkstofffreisetzung in höchstens 90 % der Zeit freisetzt, die bei einer vergleichbaren Arzneiform mit gleichem Polymerzug, jedoch ohne oder Tellneutrolisation mittels anderer Basen, dafür verstreicht.

12. Verwendung nach Anspruch 11, **dadurch gekennzeichnet, dass** die Arzneiform in Form einer multipartikulären Arzneiform, pellethaltigen Tabletten, Minitabletten, Kapseln, Sachets, Brausetabletten oder Trockensäften vorliegt.

## Claims

1. Pharmaceutical form comprising a core having an active pharmaceutical ingredient and comprising a polymer coating of a partially neutralized (meth)acrylate copolymer consisting of free-radical polymerized units of 25 to 95% by weight C₁- to C₄-alkyl esters of acrylic or of methacrylic acid and 5 to 75% by weight (meth)acrylate monomers having an anionic group, where 0.1 to 25% of the contained anionic groups are neutralized by a base, **characterized in that** the base is lysine or a cationic, organic base having a molecular weight above 150.

2. Pharmaceutical form according to Claim 1,
**characterized in that** the cationic, organic base having a molecular weight above 150 is histidine, arginine, a polyhistidine, a polyarginine, a polylysine, a phospholipid such as phosphatidylcholine, a ribonucleoside or a deoxyribonucleoside, a base from cationic surface-active excipients or emulsifiers.

3. Pharmaceutical form according to Claim 1 or 2, **characterized in that** the anionic (meth)acrylate copolymer consists of free-radical polymerized units of 40 to 60% by weight methacrylic acid and 60 to 40% by weight methyl methacrylate or 60 to 40% by weight ethyl acrylate.

4. Pharmaceutical form according to one or more of Claims 1 to 3, **characterized in that** the anionic (meth)acrylate copolymer is present in a mixture with copolymers of methyl methacrylate and/or ethyl acrylate and where appropriate less than 5% by weight methacrylic acid, copolymers of methyl methacrylate, butyl methacrylate and dimethylethyl methacrylate, copolymers of methyl methacrylate, ethyl acrylate and trimethylammoniumethyl methacrylate, copolymers of methyl methacrylate and ethyl acrylate, polyvinylpyrrolidones (PVP), polyvinyl alcohols, polyvinyl alcohol-polyethylene glycol graft copolymers (Kollicoat^{®}), starch and its derivatives, polyvinyl acetate phthalate (PVAP, Coateric^{®}), polyvinyl acetate (PVAc, Kollicoat), vinyl acetate-vinylpyrrolidone copolymer (Kollidon^{o} VA64), vinyl acetate: crotonic acid 9:1 copolymer (VAC:CRA, Kollicoat^{®} VAC), polyethylene glycols having a molecular weight above 1000 (g/mol), chitosan, a crosslinked and/or noncrosslinked polyacrylic acid, an Na alginate, and/or a pectin.

5. Pharmaceutical form according to one or more of Claims 1 to 4, **characterized in that** the polymer coating comprises lysine or arginine as neutralizing agent in combination with 5 to 25% by weight of a plasticizer.

6. Pharmaceutical form according to one or more of Claims 1 to 5, **characterized in that** a layer comprising where appropriate a binder and a cationic, organic base having a molecular weight above 150 is located between the active ingredient-containing core and the polymer coating.

7. Pharmaceutical form according to one or more of Claims 1 to 5, **characterized in that** a separating layer is applied between the core having an active pharmaceutical ingredient and the polymer coating.

8. Process for producing a pharmaceutical form according to one or more of Claims 1 to 7, comprising a core having an active pharmaceutical ingredient and comprising a polymer coating of a partially neutralized anionic (meth)acrylate copolymer, in a manner known per se by pharmaceutically customary processes such as direct compression, compression of dry, wet or sintered granules, extrusion and subsequent rounding off, wet or dry granulation or direct pelleting or by binding powders (powder layering) onto active ingredient-free beads or neutral cores (nonpareilles) or active ingredient-containing particles and by applying the polymer coating in a spray process or by fluidized bed granulation.

9. Process according to Claim 8, **characterized in that** the partially neutralized anionic (meth)acrylate copolymer is used in the form of an aqueous dispersion with a solids content of from 10 to 50 percent.

10. Process according to Claim 8, **characterized in that** the partially neutralized anionic (meth)acrylate copolymer is used in the form of a redispersible powder which has been obtained from a dispersion according to Claim 9.

11. Use of a partially neutralized anionic (meth)acrylate copolymer, consisting of free-radical polymerized units of 25 to 95% by weight C₁- to C₄-alkyl esters of acrylic or of methacrylic acid and 5 to 75% by weight (meth)acrylate monomers having an anionic group, wherein 0.1 to 25% of the contained anionic groups are neutralized by a base **characterized in that** the base is lysine or a cationic, organic base having a molecular weight above 150, as coating agent for a pharmaceutical form which, in the USP 28 release test for 2 hours at pH 1.2 and a subsequent change in the buffer to the pH of the start of active ingredient release, releases 90% of the contained active ingredient in not more than 90% of the time which elapses therefor with a comparable pharmaceutical form with the same polymer pulling but without or partial neutralization by means of other bases.

12. Use according to Claim 11, **characterized in that** the pharmaceutical form is in the form of a multiparticulate pharmaceutical form, pellet-containing tablets, minitablets, capsules, sachets, effervescent tablets or reconstitutable powders.

## Revendications

1. Forme pharmaceutique, contenant un noyau comportant une substance active pharmaceutique et un enrobage polymère à base d'un copolymère de (méth)acrylate partiellement neutralisé, constitué de motifs, polymérisés par voie radicalaire, de 25 à 95 % en poids d'esters alkyliques en C₁-C₄ de l'acide acrylique ou de l'acide méthacrylique et de 5 à 75 % en poids de monomères (méth)acrylate comportant un groupe anionique, 0,1 à 25 % des groupes anioniques contenus étant neutralisés à l'aide d'une base,
**caractérisée en ce que**
la base est la lysine ou une base organique cationique ayant une masse moléculaire supérieure à 150.

2. Forme pharmaceutique selon la revendication 1,
**caractérisée en ce que** la base organique cationique ayant une masse moléculaire supérieure à 150 est l'histidine, l'arginine, une poly-histidine, une polyarginine, une poly-lysine, un phospholipide, tel que la phosphatidylcholine, un ribonucléoside ou un désoxyribonucléoside, une base provenant d'émulsifiants ou d'adjuvants tensioactifs cationiques.

3. Forme pharmaceutique selon la revendication 1 ou 2, **caractérisée en ce que** le copolymère de (méth)acrylate anionique est constitué de motifs, polymérisés par voie radicalaire, de 40 à 60 % en poids d'acide méthacrylique et 60 à 40 % en poids de méthacrylate de méthyle ou 60 à 40 % en poids d'acrylate d'éthyle.

4. Forme pharmaceutique selon une ou plusieurs des revendications 1 à 3, **caractérisée en ce que** le copolymère de (méth)acrylate anionique est présent en mélange avec des copolymères de méthacrylate de méthyle et/ou d'acrylate d'éthyle et éventuellement moins de 5 % en poids d'acide méthacrylique, de copolymères de méthacrylate de méthyle, méthacrylate de butyle et méthacrylate de diméthyléthyle, de copolymères de méthacrylate de méthyle, acrylate d'éthyle et méthylméthacrylate de triméthylammonium, de copolymères de méthacrylate de méthyle et acrylate d'éthyle, de polyvinylpyrrolidones (PVP), de poly(alcool vinylique)s, de copolymères greffés poly(alcool vinylique)-polyéthylèneglycol (Kollicoat®), d'amidon et ses dérivés, de poly(phtalate-acétate de vinyle) (PVAP, Coateric®), de poly(acétate de vinyle) (PVAc, Kollicoat), de copolymère acétate de vinyle-vinylpyrrolidone (Kollidon® VA64), de copolymère acétate de vinyle/acide crotonique 9:1 (VAC/CFA, Kollicoat® VAC), de polyéthylèneglycols ayant une masse moléculaire supérieure à 1 000 (g/mole), d'un poly(acide acrylique) réticulé ou non réticulé, de chitosane, d'un alginate de Na et/ou d'une pectine.

5. Forme pharmaceutique selon une ou plusieurs des revendications 1 à 4, **caractérisée en ce que** l'enrobage polymère contient de la lysine ou de l'arginine en tant qu'agent de neutralisation, en association avec 5 à 25 % en poids d'un plastifiant.

6. Forme pharmaceutique selon une ou plusieurs des revendications 1 à 5, **caractérisée en ce qu'**entre le noyau contenant une substance active et l'enrobage polymère se trouve une couche, contenant éventuellement un liant et une base organique cationique ayant une masse moléculaire supérieure à 150.

7. Forme pharmaceutique selon une ou plusieurs des revendications 1 à 5, **caractérisée en ce qu'**une couche de séparation est appliquée entre le noyau comportant une substance active pharmaceutique et l'enrobage polymère.

8. Procédé pour la préparation d'une forme pharmaceutique selon une ou plusieurs des revendications 1 à 7, contenant un noyau comportant une substance active pharmaceutique et un enrobage polymère à base d'un copolymère de (méth)acrylate anionique partiellement neutralisé, d'une façon connue en soi, au moyen de procédés usuels en pharmacie, tels que compression directe, compression de granulés secs, humides ou frittés, extrusion et arrondissement subséquent, granulation par voie humide ou sèche ou pastillage direct ou par fixation de poudres (powder layering) sur des noyaux neutres ou des sphères sans substance active (nonpareilles) ou des particules contenant une substance active et par application de l'enrobage polymère dans le procédé de pulvérisation ou par granulation en lit fluidisé.

9. Procédé selon la revendication 8, **caractérisé en ce que** le copolymère de (méth)acrylate anionique partiellement neutralisé est utilisé sous forme d'une dispersion aqueuse à teneur en matière solide de 10 à 50 %.

10. Procédé selon la revendication 8, **caractérisé en ce que** le copolymère de (méth)acrylate anionique partiellement neutralisé est utilisé sous forme d'une poudre redispersable, qui a été obtenue à partir d'une dispersion selon la revendication 9.

11. Utilisation d'un copolymère de (méth)acrylate anionique partiellement neutralisé, constitué de motifs, polymérisés par voie radicalaire, de 25 à 95 % en poids d'esters alkyliques en C₁-C₄ de l'acide acrylique ou de l'acide méthacrylique et de 5 à 75 % en poids de monomères (méth)acrylate comportant un groupe anionique, 0,1 à 25 % des groupes anioniques contenus étant neutralisés à l'aide d'une base,
**caractérisée en ce que**
la base est la lysine ou une base organique cationique ayant une masse moléculaire supérieure à 150, en tant qu'enrobage pour une forme pharmaceutique qui libère 90 % de la substance active contenue, dans l'essai de libération selon USP 28 pendant 2 heures à pH 1,2 et re-tamponnage subséquent au pH du début de la libération de substance active, en au maximum 90 % du temps qui s'étend pour cela dans le cas d'une forme pharmaceutique comparable comportant le même enrobage polymère, mais sans neutralisation partielle ou avec neutralisation partielle à l'aide d'autres bases.

12. Utilisation selon la revendication 11,
**caractérisée en ce que** la forme pharmaceutique se trouve sous forme d'une forme pharmaceutique multiparticulaire, de comprimés contenant des granules, de mini-comprimés, de gélules, de sachets, de comprimés effervescents ou de poudres pour redissolution.
